# EUROPEAN PATENT SPECIFICATION

(11) **EP 1 833 369 B1**
(45) Date of publication and mention of the grant of the patent: **18.03.2009**
(21) Application number: 04775353.8
(22) Date of filing: 31.08.2004
(51) Int. Cl.: A61B 5/11

(54) **APPARATUS FOR DETECTING DIASTOLIC HEART FAILURE**
GERÄT ZUM NACHWEIS VON DIASTOLISCHER HERZINSUFFIZIENZ
APPAREIL DE DETECTION DE L'INSUFFISANCE CARDIAQUE DIASTOLIQUE

(43) Date of publication of application: 19.09.2007
(73) Proprietor: St. Jude Medical AB, 175 84 Järfälla (SE)
(72) Inventor: HEDBERG, Sven-Erik, S-196 32 Kungsängen (SE)
(86) International application number: PCT/SE2004/001247
(87) International publication number: WO 2006/025770

(56) References cited:
- WO-A1-03/061473
- US-A- 6 009 349
- US-A1- 2003 083 702
- REDFIELD M.M. ET AL: 'Burden of Systolic and Diastolic Ventricular Dysfunction in the Community' JAMA vol. 289, no. 2, 08 January 2003, pages 194 - 202, XP002986108
- BRUCH C. ET AL: 'Doppler Tissue Analysis of Mitral Annular Velocities: Evidence for Systolic Abnormalities in Patients with Diastolic Heart Failure' JOURNAL OF THE AMERICAN SOCIETY OF ECHOCARDIOGRAPHY vol. 16, no. 10, October 2003, pages 1031 - 1036, XP002986109

## Description

### Technical Field

The present invention relates to an implantable medical apparatus for detecting diastolic heart failure, DHF, and a pacemaker comprising said apparatus.

### Background of the Invention

There is a growing recognition that congestive heart failure caused by a predominant abnormality in the diastolic function, i.e. diastolic heart failure, DHF, is both common and causes significant morbidity and mortality. Therefore early detection of DHF is important. Patients do not, however, seem to have symptoms at an early stage. In addition it has been hard to separate diastolic and systolic heart failure, and they may also exist simultaneously.

DHF is characterized by a slowing down of the recoil effect during early diastole, i.e. during the isovolumic ventricular relaxation and the rapid left ventricular filling phase, before the atrial contraction. This has been observed by measuring the velocity of the mitral annulus. According to an article by Margaret M. Redfield et. al., "Burden of Systolic and Diastolic Ventricular Dysfunction in the Community, JAMA, Vol. 289, No. 2, p. 194-202, January 8, 2003, the velocity of the mitral annulus motion reflects the state of DHF. The article shows that the velocity of the mitral annulus motion decreases when the diastolic function gets more deteriorated.

However, the measurement of heart tissue has already been used for other purposes. For example, US 5 480 412 A discloses a processing system and method for deriving an improved hemodynamic indicator from cardiac wall acceleration signals. The cardiac wall acceleration signals are provided by a cardiac wall sensor that responds to cardiac mechanical activity. The cardiac wall acceleration signals are integrated over time to derive cardiac wall velocity signals, which are further integrated over time to derive cardiac wall displacement signals. The cardiac wall displacement signals correlate to known hemodynamic indicators. An implantable cardiac stimulating device using cardiac wall displacement signals to detect and discriminate cardiac arrhythmias is also described. Further, US 5 628 777 A discloses an implantable lead comprising an accelerometer-based cardiac wall motion sensor. Said sensor transduces accelerations of cardiac tissue to provide electrical signals indicative of cardiac wall motion to an implantable cardiac stimulation device. Said device uses said electrical signals to detect and discriminate among potentially malignant cardiac arrhythmias. Furthermore, US 6 009 349 A discloses a processing system for an implantable cardiac device, said device having cardiac wall accelerator sensors for providing a cardiac wall accelerator signal as a function of cardiac wall contractile motion, the sensors being positioned in the right atrium and right ventricle of a patent's heart.

### The Object of the Invention

The object of the present invention is to utilize above mentioned knowledge to propose a technique for detecting DHF, preferably at an early stage when the patient still does not seem to have any symptoms, based on the movement of the mitral annulus.

### Summary of the Invention

The above-mentioned object is achieved by an apparatus and a pacemaker, of the kind mentioned in the introductory portion of the description and having the characterizing features of claims 1 and 16, respectively. It has been shown that the movement of the valve plane of the heart is comparable to the movement of the mitral annulus, so by the apparatus, pacemaker, of the present invention is an efficient technique for detecting DHF provided, also for detecting DHF at an early stage when the patient still does not seem to have any symptoms.

According to advantageous embodiments of the apparatus according to the present invention, the analysing means comprise a comparison means for comparing the measured movement of the valve plane with predetermined reference values, and the measuring means is arranged to measure the movement of the valve plane during early diastole, before the atrial contraction.

According to another advantageous embodiment of the apparatus according to the present invention, the apparatus comprises first detection means for detecting the T-wave, and the measuring means is arranged to measure the movement of the valve plane in the vicinity of the T-wave.

According to a further advantageous embodiment of the apparatus according to the present invention, the apparatus comprises second detection means for detecting the QRS complex, and the measuring means is arranged to measure the movement of the valve plane during a time window starting just after the QRS complex and ending before the atrial contraction.

According to an advantageous embodiment of the apparatus according to the present invention, the apparatus comprises activity measuring means for measuring the condition of the patient, and the measuring means is arranged to measure the movement of the valve plane when the activity measuring means indicates resting conditions of the patient.

According to a further advantageous embodiment of the apparatus according to the present invention, the measuring means is arranged to measure movement of the valve plane by measuring the velocity or the acceleration of the valve plane.

According to other advantageous embodiments of the apparatus according to the present invention, the measuring means comprises an accelerometer arranged to be placed on or close to the valve plane, and the apparatus comprises calculating means for calculating the velocity of the valve plane by summing-up or integrating the measured acceleration values.

According to still other advantageous embodiments of the apparatus according to the present invention, the measuring means is arranged to measure the pressure from the inner walls of the coronary sinus, the great cardiac vein or a coronary vein, said pressure correlating with the movement of the valve plane, and the measuring means arranged to measure said pressure comprises a pressure sensor with a circumferential sensitivity arranged to be placed in the coronary sinus, the great cardiac vein or in a coronary vein.

According to yet other advantageous embodiments of the apparatus according to the present invention, the analysing means are arranged to find the peak value from measured values from one heart interval, the apparatus comprises an averaging means for forming an average value of peak values from measured values from several heart intervals, and the apparatus comprises storing means for storing measured values together with the time of occurrence, for later analysis.

### Brief Description of the Drawings

The present invention will now be described, for exemplary purposes, in more detail by way of embodiments and with reference to the enclosed drawings, in which:
- Fig. 1: shows diagrams from the mentioned article by Margaret M. Redfield et. al., showing the velocity of the mitral annulus during early diastole in relation to the degree of DHF,
- Fig. 2: shows a schematic block diagram of an embodiment of a pace- maker according to the present invention, and
- Fig. 3: shows a flow chart illustrating an embodiment of the method ac- cording to the present invention.

### Detailed Description of Preferred Embodiments

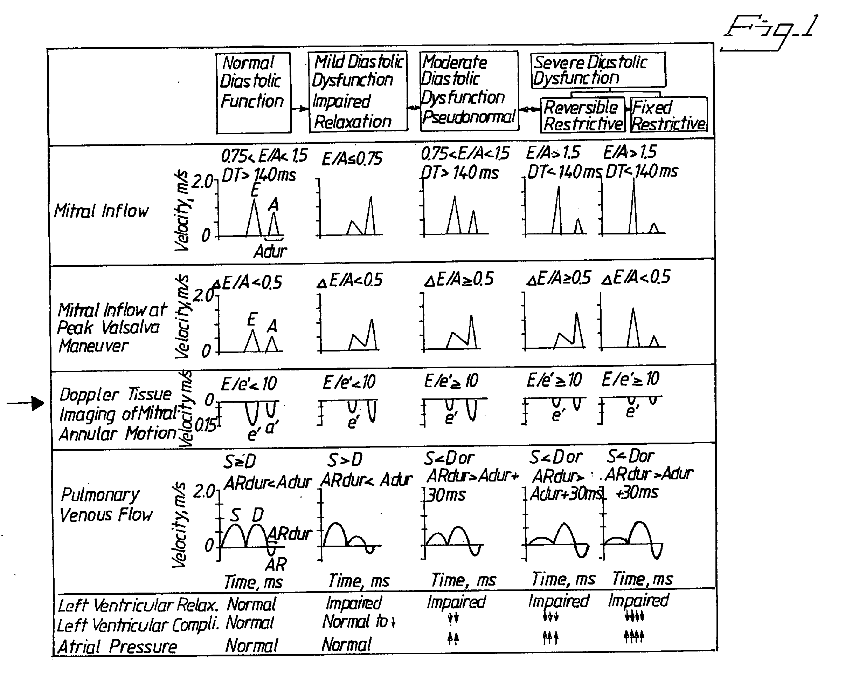

In the diagrams for Doppler Tissue Imaging of Mitral Annular Motion of fig. 1, the curve e' shows the velocity of mitral annulus motion during early diastole. It is clear from the diagrams that the velocity of the mitral annulus motion decreases when the diastolic dysfunction gets more severe.

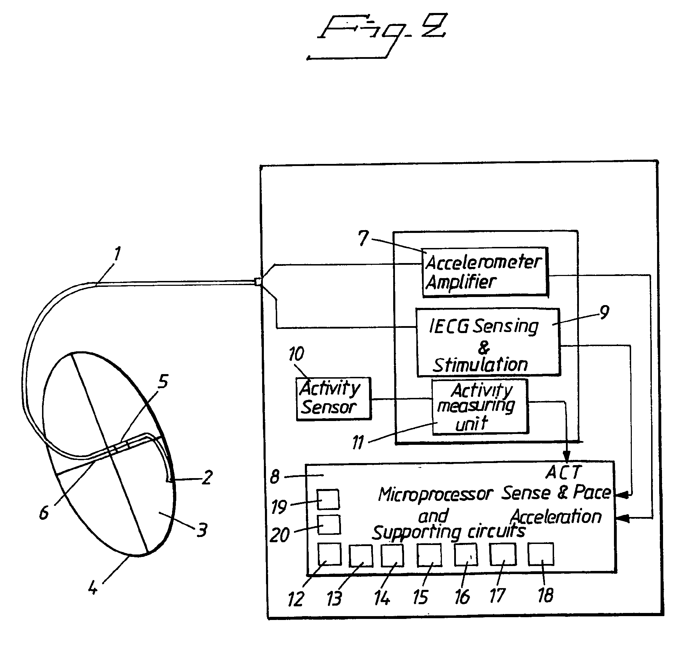

Fig. 2 shows an embodiment of a pacemaker comprising an apparatus according to the present invention. The pacemaker is adapted for left ventricular pacing only, and a left ventricular lead 1 of the pacemaker is with its electrode 2 connected to the left ventricle 3 of a patent's heart 4. Integrated with the lead 1 is an accelerometer 5 which is placed in the valve plane 6 of the patent's heart 4, but can also be placed close to the valve plane 6 in the lower part of the right atrium, or inside one of the ventricles. The accelerometer 5 is arranged to measure the acceleration of the valve plane 6 and is via the lead 1 connected to an accelerometer amplifier 7 arranged to amplify the acceleration signals, which in turn is connected to microprocessor and supporting circuits 8 of the pacemaker. The electrode 2 of the lead 1 is connected to IECG sensing and stimulation means 9 which in turn is connected to the microprocessor and supporting circuits 8.

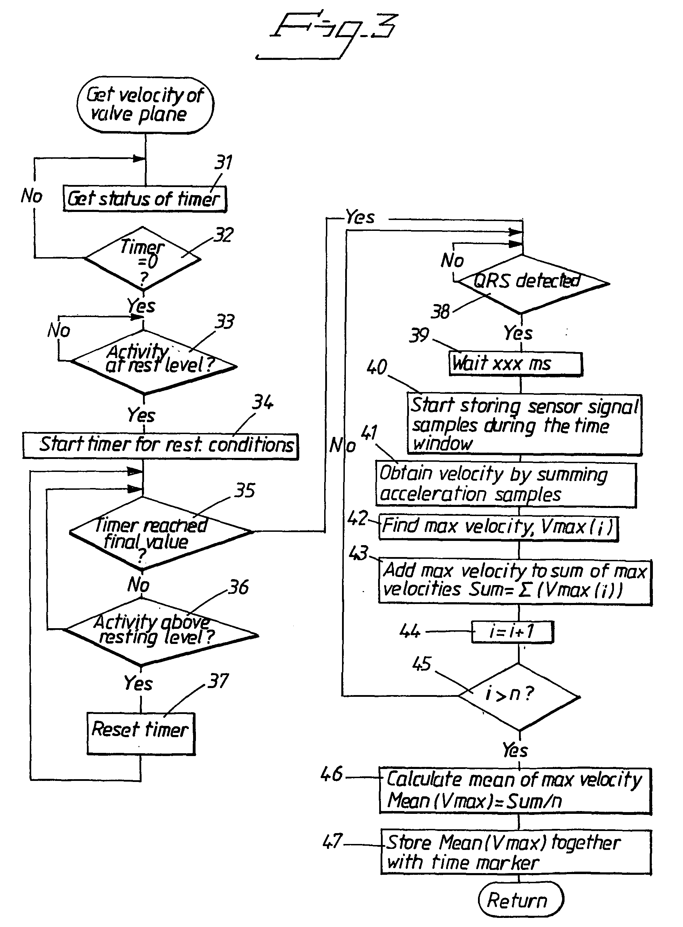

The DHF is a slow process. Therefore, the points in time for measuring the acceleration of the valve plane 6 is preferably applied to occasions when the patient is only making small movements and the signal interference is low, e.g. during sleep. In order to identify such moments the pacemaker comprises an activity sensor 10 connected to an activity measuring unit 11 for measuring the condition of the patient, which unit 11 in turn is connected to the microprocessor and supporting circuits 8. The microprocessor and supporting circuits 8 provide a timer 12 for starting the process of measuring the movement of the valve plane 6, the function of which is described in connection to Fig. 3.

The diastolic phase of interest is during the isovolumic ventricular relaxation and the rapid left ventricular filling phase, before the atrial contraction, thus the microprocessor and supporting circuits 8 provide detection means 13 for detecting the QRS complex from the signals captured by the electrode 2. The occurrence of peak velocity of the valve plane 6 takes place only a small varying time delay after the QRS complex. The microprocessor and supporting circuits 8 are arranged to lay down a time window, with a width of about 100 ms, enough to cover the valve plane motion during the relaxation phase, which starts just after the QRS complex and ends before the atrial contraction, and the accelerometer 5 is arranged to measure the acceleration of the valve plane 6 during said time window. The microprocessor and supporting circuits 8 provide calculating means 14 for calculating the velocity of the valve plane 6 by summing-up or integrating the measured acceleration values during said time window. The microprocessor and supporting circuits 8 also provide analysing means 15 for finding the peak value from measured values from one heart interval. Further, the microprocessor and supporting circuits 8 provide storing means 16 for storing measured values together with the time of occurrence, for later analysis. Thus, the development of valve plane movement values over time can be obtained. Additionally, the microprocessor and supporting circuits 8 provide averaging means 17 for forming an average value of peak values from measured values from several heart intervals. The analysing means 15 are arranged to analyse the measurement of the movement of the valve plane 6, and comprise a determining means 18 for determining a slowdown of the movement of the valve plane 6 for indicating a DHF state of the heart 4 of a patient from the determined slowdown. Further, the analysing means 15 comprise a comparison means 19 for comparing the measured movement of the valve plane 6 with predetermined reference values. The comparison with reference values supports the indication of a DHF state. Finally, the microprocessor and supporting circuits 8 provide control means 20 for optimising pacing therapy depending on the result of the analysis of the measured movement of the valve plane 6.

If the implantation of the pacemaker occurs at a point in time when no essential DHF is at hand, the peak velocity obtained will be the basis for evaluating the degree of DHF. The pacemaker can also measure absolute peak velocity if the processed accelerometer signal is calibrated. This can be done by comparing the peak velocity found by the pacemaker with the peak velocity found by ultrasonic equipment suitable for such measurements. It is enough to measure and calibrate one peak velocity, since the accelerometer will show zero signal at zero velocity.

Fig. 3 shows a flow chart illustrating a method of detecting DHF. Since velocity measurement of the valve plane should be carried out during resting condition of the patient, e.g. during sleep, the pacemaker comprises a timer which starts the process of measuring the movement of the valve plane. First the status of the timer is checked, at 31. If the timer has counted down to zero, at 32, the next step is to wait for a resting period, at 33, long enough to ensure resting condition of the patient. When a resting period occurs, the timer starts, at 34. If the activity of the patient is not above resting level, at 36 and when the timer reaches its final value, at 35, the detection of the QRS complex starts, at 38. If the activity of the patient is above resting level, at 36, the timer is reset, at 37.

When the QRS complex is detected, a delay is laid out, at 39, at the end of which the time window is opened, and the storing of accelerator signal samples during the time window starts, at 40. The acceleration samples from the accelerator are integrated or summed up, at 41, to obtain the velocity, and the peak velocity is found during said time window, at 42, and added to a sum of peak velocities, at 43. The steps 38 to 44 are repeated for n heart intervals, and when peak velocities from n heart intervals have been collected, at 45, and added to the sum of peak velocities, at 43, an average value of the peak velocities from the n heart intervals is formed, at 46, by dividing the sum of peak velocities by the number of heart intervals, n. The number n may be in the order of 10, but it is not a critical number. The average value of the peak velocities is stored together with the time of occurrence, at 47, for analysis in order to detect a DHF state of the heart of a patient. Said analysis comprises the step of determining a slowdown of the movement of the valve plane for indicating a DHF state of the heart of a patient from the determined slowdown.

## Claims

1. An implantable medical apparatus for detecting diastolic heart failure, DHF, **characterized in that** the apparatus comprises a measuring means for measuring the movement of the valve plane of the heart, and analysing means for analysing the measurement of the movement of the valve plane comprising a determining means for determining a slowdown of the movement of the valve plane for indicating a DHF state of the heart of a patient from the determined slowdown.

2. An apparatus according to claim 1, **characterized in that** the analysing means comprise a comparison means for comparing the measured movement of the valve plane with predetermined reference values.

3. An apparatus according to claim 1 or 2, **characterized in that** the measuring means is arranged to measure the movement of the valve plane during early diastole, before the atrial contraction.

4. An apparatus according to claim 3, **characterized in that** the apparatus comprises first detection means for detecting the T-wave, and the measuring means is arranged to measure the movement of the valve plane in the vicinity of the T-wave.

5. An apparatus according to claim 3 or 4, **characterized in that** the apparatus comprises second detection means for detecting the QRS complex, and the measuring means is arranged to measure the movement of the valve plane during a time window starting just after the QRS complex and ending before the atrial contraction.

6. An apparatus according any of the claims 1-5, **characterized in that** the apparatus comprises activity measuring means for measuring the condition of the patient, and the measuring means is arranged to measure the movement of the valve plane when the activity measuring means indicates resting conditions of the patient.

7. An apparatus according to any of the claims 1-6, **characterized in that** the measuring means is arranged to measure the velocity of the valve plane.

8. An apparatus according to any of the claims 1-6, **characterized in that** the measuring means is arranged to measure the acceleration of the valve plane.

9. An apparatus according to claim 8, **characterized in that** the measuring means comprises an accelerometer arranged to be placed on or close to the valve plane.

10. An apparatus according to claim 8 or 9, **characterized in that** the apparatus comprises calculating means for calculating the velocity of the valve plane by summing-up or integrating the measured acceleration values.

11. An apparatus according to any of the claims 1-6, **characterized in that** the measuring means is arranged to measure the pressure from the inner walls of the coronary sinus, the great cardiac vein or a coronary vein, said pressure correlating with the movement of the valve plane.

12. An apparatus according to claim 11, **characterized in that** the measuring means arranged to measure said pressure comprises a pressure sensor with a circumferential sensitivity arranged to be placed in the coronary sinus, the great cardiac vein or in a coronary vein.

13. An apparatus according to any of the claims 1-12, **characterized in that** the analysing means are arranged to find the peak value from measured values from one heart interval.

14. An apparatus according to claims 13, **characterized in that** the apparatus comprises an averaging means for forming an average value of peak values from measured values from several heart intervals.

15. An apparatus according to any of the claims 1-14, **characterized in that** the apparatus comprises storing means for storing measured values together with the time of occurrence, for later analysis.

16. A pacemaker, **characterized in that** the pacemaker comprises an apparatus according to any one of the preceding claims, and control means for optimising pacing therapy depending on the result of the analysis of the measured movement of the valve plane.

## Patentansprüche

1. Implantierbare medizinische Vorrichtung zum Detektieren einer diastolischen Herzinsuffizienz DHF, **dadurch gekennzeichnet, dass** das Gerät eine Messvorrichtung zum Messen der Bewegung der Klappenebene des Herzens und eine Analysevorrichtung enthält, zum Analysieren des Maßes der Bewegung der Klappenebene, enthaltend eine Bestimmungsvornchtung zum Bestimmen einer Verlangsamung der Bewegung der Klappenebene zur Anzeige eines DHF-Zustandes des Herzens eines Patienten aus der festgestellten Verlangsamung.

2. Vorrichtung nach Anspruch 1, **dadurch gekennzeichnet, dass** die Analysevorrichtung eine Vergleichsvorrichtung zum Vergleichen der gemessenen Bewegung der Klappenebene mit vorbestimmten Referenzwerten enthält.

3. Vorrichtung nach Anspruch 1 oder 2, **dadurch gekennzeichnet, dass** die Messvorrichtung ausgelegt ist, die Bewegung der Klappenebene während der vorzeitigen Diastole vor der atrialen Kontraktion zu messen.

4. Vorrichtung nach Anspruch 3, **dadurch gekennzeichnet, dass** das Gerät eine erste Detektionsvorrichtung zum Detektieren der T-Welle enthält und die Messvorrichtung ausgelegt ist, die Bewegung der Klappenebene benachbart zur T-Welle zu messen.

5. Vorrichtung nach Anspruch 3 oder 4, **dadurch gekennzeichnet, dass** das Gerät eine zweite Detektionsvorrichtung zum Detektieren des QRS-Komplexes enthält und die Messvorrichtung ausgelegt ist, die Bewegung der Klappenebene während eines Zeitfensters zu messen, das unmittelbar nach dem QRS-Komplex beginnt und vor der atrialen Kontraktion endet.

6. Vorrichtung nach einem der Ansprüche 1 bis 5, **dadurch gekennzeichnet, dass** das Gerät eine Aktivitätsmessvornchtung zum Messen des Zustandes des Patienten enthält und die Messvorrichtung ausgelegt ist, die Bewegung der Klappenebene zu messen, wenn die Aktivitätsmessvornchtung Ruhezustände des Patienten anzeigt.

7. Vorrichtung nach einem der Ansprüche 1 bis 6, **dadurch gekennzeichnet, dass** die Messvorrichtung ausgelegt ist, die Geschwindigkeit der Klappenebene zu messen.

8. Vorrichtung nach einem der Ansprüche 1 bis 6, **dadurch gekennzeichnet, dass** die Messvorrichtung ausgelegt ist, die Beschleunigung der Klappenebene zu messen.

9. Vorrichtung nach Anspruch 8, **dadurch gekennzeichnet, dass** die Messvorrichtung einen Beschleunigungsmesser enthält, der ausgelegt ist, an oder nahe der Klappenebene positioniert zu werden.

10. Vorrichtung nach Anspruch 8 oder 9, **dadurch gekennzeichnet, dass** das Gerät eine Berechnungsvorrichtung zum Berechnen der Geschwindigkeit der Klappenebene durch Summieren oder Integrieren der gemessenen Beschleunigungswerte enthält.

11. Vorrichtung nach einem der Ansprüche 1 bis 6, **dadurch gekennzeichnet, dass** die Messvorrichtung ausgelegt ist, den Druck von den inneren Wänden des koronaren Sinus, der großen Herzvene oder einer koronaren Vene zu messen, wobei der genannte Druck mit der Bewegung der Klappenebene korreliert.

12. Vorrichtung nach Anspruch 11, **dadurch gekennzeichnet, dass** die zum Messen des genannten Druckes ausgelegte Messvorrichtung einen Drucksensor mit einer peripheren Empfindlichkeit enthält, der ausgelegt ist, im koronaren Sinus, in der großen Herzvene oder in einer koronaren Vene positioniert zu werden.

13. Vorrichtung nach einem der Ansprüche 1 bis 12, **dadurch gekennzeichnet, dass** die Analysevorrichtung ausgelegt ist, den Spitzenwert von den gemessenen Werten aus einem Herzintervall zu finden.

14. Vorrichtung nach Anspruch 13, **dadurch gekennzeichnet, dass** das Gerät eine Mittelwertbildungsvorrichtung zum Bilden eines Mittelwerts der Spitzenwerte von den gemessenen Werten aus mehreren Herzintervallen enthält.

15. Vorrichtung nach einem der Ansprüche 1 bis 14, **dadurch gekennzeichnet, dass** das Gerät eine Speichervorrichtung zum Speichern gemessener Werte zusammen mit der Zeit ihres Auftretens für eine spätere Analyse enthält.

16. Schrittmacher, **dadurch gekennzeichnet, dass** der Schrittmacher eine Vorrichtung nach einem der vorhergehenden Ansprüche und eine Steuervorrichtung enthält zum Optimieren der Stimulationstherapie abhängig von dem Ergebnis der Analyse der gemessenen Bewegung der Klappenebene.

## Revendications

1. Dispositif médical implantable de détection d'une insuffisance cardiaque diastolique, DHF, **caractérisé en ce que** le dispositif comprend un moyen de mesure du mouvement du plan de la valvule du coeur et un moyen d'analyse pour analyser la mesure du mouvement du plan de la valvule, comprenant un moyen de détermination pour déterminer un ralentissement du mouvement du plan de la valvule pour indiquer un état de DHF du coeur d'un patient à partir du ralentissement déterminé.

2. Dispositif suivant la revendication 1, **caractérisé en ce que** le moyen d'analyse comprend un moyen de comparaison, pour comparer le mouvement mesuré du plan de la valvule à des valeurs témoins déterminées à l'avance.

3. Dispositif suivant la revendication 1 ou 2, **caractérisé en ce que** le moyen de mesure est conçu pour mesurer le mouvement du plan de la valvule pendant une diastole précoce avant la contraction de l'oreillette.

4. Dispositif suivant la revendication 3, **caractérisé en ce que** le dispositif comprend un premier moyen de détection, pour détecter l'onde T et le moyen de mesure est conçu pour mesurer le mouvement du plan de la valvule à proximité de l'onde T.

5. Dispositif suivant la revendication 3 ou 4, **caractérisé en ce que** le dispositif comprend un deuxième moyen de détection, pour détecter le complexe QRS et le moyen de mesure est conçu pour mesurer le mouvement du plan de la valvule pendant un créneau temporel débutant juste après le complexe QRS et se terminant avant la contraction de l'oreillette.

6. Dispositif suivant l'une quelconque des revendications 1 à 5, **caractérisé en ce que** le dispositif comprend un moyen de mesure de l'activité, pour mesurer l'état du patient et le moyen de mesure est conçu pour mesurer le mouvement du plan de la valvule lorsque le moyen de mesure de l'activité indique des conditions de repos du patient.

7. Dispositif suivant l'une quelconque des revendications 1 à 6, **caractérisé en ce que** le moyen de mesure est conçu pour mesurer la vitesse du plan de la valvule.

8. Dispositif suivant l'une quelconque des revendications 1 à 6, **caractérisé en ce que** le moyen de mesure est conçu pour mesurer l'accélération du plan de la valvule.

9. Dispositif suivant la revendication 8, **caractérisé en ce que** le moyen de mesure comprend un accéléromètre conçu pour être placé sur ou près du plan de la valvule.

10. Dispositif suivant les revendications 8 ou 9, **caractérisé en ce que** le dispositif comprend un moyen de calcul, pour calculer la vitesse du plan de la valvule en sommant ou en intégrant les valeurs d'accélération qui sont mesurées.

11. Dispositif suivant l'une quelconque des revendications 1 à 6, **caractérisé en ce que** le moyen de mesure est conçu pour mesurer la pression à partir des parois intérieures du sinus coronaire, de la grande veine cardiaque ou d'une veine coronaire, cette pression étant corrélée au mouvement du plan de la valvule.

12. Dispositif suivant la revendication 11, **caractérisé en ce que** le moyen de mesure, conçu pour mesurer la pression,comprend un capteur de pression, ayant une sensibilité circonférentielle et destiné à être placé dans le sinus coronaire, la grande veine cardiaque ou dans une veine coronaire.

13. Dispositif suivant l'une quelconque des revendications 1 à 12, **caractérisé en ce que** le moyen d'analyse est conçu pour trouver la valeur de crête à partir des valeurs mesurées dans un intervalle cardiaque.

14. Dispositif suivant la revendication 13, **caractérisé en ce que** le dispositif comprend un moyen pour faire une moyenne, pour former une valeur moyenne des valeurs de crête à partir des valeurs mesurées dans plusieurs intervalles cardiaques.

15. Dispositif suivant l'une quelconque des revendications 1 à 14, **caractérisé en ce que** le dispositif comprend des moyens de mémorisation, pour mémoriser des valeurs mesurées ensemble avec l'instant où elles se produisent, en vue d'une analyse ultérieure.

16. Stimulateur cardiaque, **caractérisé en ce que** le stimulateur cardiaque comprend un dispositif suivant l'une des revendications précédentes et des moyens de commande, pour optimiser la thérapie d'entraînement en fonction du résultat d'analyse du mouvement mesuré du plan de la valvule.
